# EUROPÄISCHE PATENTSCHRIFT

(11) **EP 1 038 547 B1**
(45) Veröffentlichungstag und Bekanntmachung des Hinweises auf die Patenterteilung: **29.09.2004**
(21) Anmeldenummer: 00104032.8
(22) Anmeldetag: 26.02.2000
(51) Int. Cl.: A61N 1/05

(54) **Dilatierbare Herzelektrodenanordnung zur Implantation insbesondere im Koronarsinus des Herzens**
Expandable cardiac lead for implantation in the coronary sinus
Electrode cardiaque extensible destinée à être implantée dans le sinus coronaire

(30) Priorität: 20.03.1999 DE 19912635
(43) Veröffentlichungstag der Anmeldung: 27.09.2000
(73) Patentinhaber: BIOTRONIK Mess- und Therapiegeräte GmbH & Co Ingenieurbüro Berlin, 12359 Berlin (DE)
(72) Erfinder: Rexhausen, Hermann, 12359 Berlin (DE); Jordaens, Luc, Prof., 3071-AG-Rotterdam (NL)
(74) Vertreter: Hübner, Gerd, Dipl.-Phys.

(56) Entgegenhaltungen:
- WO-A-98/42403
- US-A- 5 170 802
- US-A- 5 755 765

## Beschreibung

Die Erfindung betrifft eine dilatierbare Herzelektrodenanordnung zur Implantation insbesondere im Koronarsinus des Herzens.

Zum Hintergrund der Erfindung ist grundsätzlich festzuhalten, daß Herzschrittmacher und Defibrillatoren mit ihren Elektrodenanordnungen in der Regel auf die rechten Herzkammern zugreifen. Dies ist einerseits bedingt durch die bessere Zugänglichkeit der rechten Herzkammern für entsprechende Elektrodenkatheter, anderseits liegt das Sinusknotengewebe als natürlicher Schrittmacher des gesunden Herzens ebenfalls im rechten Herzbereich, nämlich im Bereich des rechten Vorhofes.

Die linken Herzkammern sind für herkömmliche transvenöse Elektroden-Implantations-Prozeduren hingegen schlecht zugänglich. Da sich die Erregung des Herzmuskelgewebes jedoch automatisch auch auf die jeweils andere Herzseite ausbreitet, sind Messung und Stimulation über in den rechten Herzkammern implantierte Elektrodenanordnungen in der Regel ausreichend.

Allerdings versorgen die rechten Herzkammern lediglich den volumenmäßig kleineren Lungen-Blutkreislauf und sind dementsprechend geringer dimensioniert als die linken Herzkammern. An der gesamten Herzpumpleistung sind sie folglich nur zu einem kleineren Teil beteiligt. In kardiologischer Hinsicht besteht nun aufgrund der unterschiedlichen Größe und Bedeutung von rechter und linker Herzhälfte ein grundsätzliches Interesse daran, auch auf die linke Herzseite einen direkten Zugriff durch entsprechende Elektrodenanordnungen zu erlauben. Damit kann beispielsweise der Zeitablauf der Erregungsausbreitung von der rechten zur linken Herzseite festgestellt werden. Ferner sind bestimmte Formen tachykarder Herzrythmusstörungen mit entsprechenden Elektrodenanordnungen auf der linken Herzseite bekämpfbar, wie beispielsweise Störungen, bei denen die Erregung des Herzmuskelgewebes im Kreis den linken Vorhof, beide Herzkammern, den rechten Vorhof, den linken Vorhof usw. durchläuft. Solche tachykarden Herzrythmusstörungen können nämlich frühzeitig durch die damit verbundenen überlangen Reizleitungszeiten zwischen linkem und rechtem Vorhof erkannt werden.

Typischerweise können der linke Vorhof des Herzens und - mit Einschränkungen - auch die linke Herzkammer durch Elektrodenanordnungen erreicht werden, die in den Koronarsinus auf der linken Seite des Herzens implantiert werden. Hierzu sind im Stand der Technik bereits verschiedene Vorschläge gemacht worden.

So ist aus der US-A-5,423,772 eine Schrittmacherelektrode mit drei unterschiedlich steifen Abschnitten, nämlich einem verstärkten Hauptabschnitt, einer Zwischenzone und einem weichen Spitzenabschnitt bekannt. Das in den Koronarsinus einführbare Ende dieses Katheters weist ferner zwei Krümmungsbereiche mit einem größeren Krümmungsradius vor der Spitze und einem kleineren Krümmungsradius an der Spitze auf. Die erstgenannte Biegung sorgt für eine mechanische Fixierung der Elektrode im Koronarsinus, während das hakenförmige Ende den Winkel und die Andruckkraft einer an der Spitze des Katheters angeordneten Tipelektrode zur Wand des Koronarsinus verbessert.

Bezüglich der Elektrodenbelegung zeigt die US-A-5,423,772 neben der Tipelektrode lediglich weitere etwa gleich große Ringelektroden, deren Anzahl bis zehn oder mehr angegeben wird. Die Elektrodenbelegung soll sich dabei nach den jeweiligen Meßzwecken der Elektrodenanordnung richten.

Eine Problematik bei der Implantation von Elektrodenkathetern insbesondere im Koronarsinus liegt darin, daß der implantierte Katheter zu einer Verengung des Gefäßquerschnittes und damit zu einer Einschränkung der Blutströmung führt, was gerade bei der Versorgung des Herzmuskels von erheblichem Nachteil ist.

Zur Lösung dieser Problematik schlägt die US-A-5,170,802 eine dilatierbare Elektrodenanordnung zur Implantation insbesondere im Koronarsinus des Herzens vor, bei der eine oder mehrere stentartig aufweitbare Elektroden vorgesehen sind, die mit jeweils individuellen Zuleitungen oder einem gemeinsamen Sammelanschluß gekoppelt sind. Die Dilatation der Elektrodenanordnung kann - wie von Gefäßwandstützen oder Stents" bekannt - durch einen Ballonkatheter oder durch eine federelastische Ausführung der Elektrodenstruktur erfolgen. Konkret ist in der US-A-5,170,802 im übrigen eine Defibrillationselektrode offenbart.

Schließlich ist aus der US-A-5,531,779 eine stentartige Defibrillationselektrode bekannt, deren Elektrodenkörper aus einzelnen, im expandierten Zustand eine Art geschlossenen Korb bildenden Drähten besteht. Die Enden dieser Drähte sind durch ring- oder kappenförmige Aufsätze aus elektrisch leitfähigem Material zusammengefaßt und dadurch mechanisch fixiert. Über eine der beiden Endkappen wird dabei die Anschlußleitung der Elektrode elektrisch angebunden.

Der Erfindung liegt nun die Aufgabe zugrunde, stentartig dilatierbare Herzelektrodenanordnungen zur Implantation insbesondere im Koronarsinus des Herzens in ihrer Funktionalität zu verbessern und insbesondere eine kombinierte Defibrillator- und Schrittmacherelektrode für den Koronarsinus zu schaffen.

Die Lösung dieser Aufgabe ist durch die Merkmale des Patentanspruches 1 gegeben. Demnach sind einerseits eine stentartige Defibrillatorelektrode mit einer aufweitbaren, elektrisch leitfähigen Struktur und andererseits zwei Schrittmacherelektroden vorgesehen, die gegenüber der Defibrillationselektrode eine kleinere Oberfläche aufweisen, an den einander abgewandten Enden der Defibrillatorelektrode angeordnet sind und ebenfalls eine stentartig aufweitbare, elektrisch leitfähige Struktur aufweisen. Zwischen den Schrittmacherelektroden einerseits und der Defibrillatorelektrode andererseits sind jeweils Isolationszonen vorgesehen. Diese dienen zur elektrischen Trennung der angegebenen Elektroden voneinander und stellen darüber hinaus die mechanische Verbindung zwischen den einzelnen Elektroden her. Schließlich sind für die Defibrillator- und Schrittmacherelektroden elektrische Zuleitungen vorgesehen. Unter dem Begriff "ringabschnittartig" ist im übrigen eine geteilte Ringelektrode zu verstehen, bei der nur Umfangsabschnitte als Meßelektrode dienen.

Durch den erfindungsgemäßen Aufbau einer Herzelektrodenanordnung können Elektrodenimplantate mit kombinierter Defibrillator- und Schrittmacher-Funktionalität auch im Bereich der linken Herzseite bei vertretbarem Implantationsaufwand eingesetzt werden.

Bevorzugte Ausführungsformen einer solchen Herzelektrodenanordnung sind im übrigen in den Unteransprüchen angegeben.

Ausführungsbeispiele der Erfindung werden in der nachfolgenden Beschreibung anhand der beigefügten Zeichnungen näher erläutert. Es zeigen:
- Fig. 1: eine ausschnittsweise Abwicklung einer dilatierbaren Herzelektrodenanordnung in einer ersten Ausführungsform,
- Fig. 2: einen Teilschnitt der Anordnung gemäß der Schnittlinie II-II nach Fig. 1,
- Fig. 3: eine ausschnittsweise Abwicklung einer dilatierbaren Herzelektrodenanordnung in einer zweiten Ausführungsform, und
- Fig. 4: einen Teilschnitt der Anordnung gemäß Schnittlinie IV-IV nach Fig. 3.

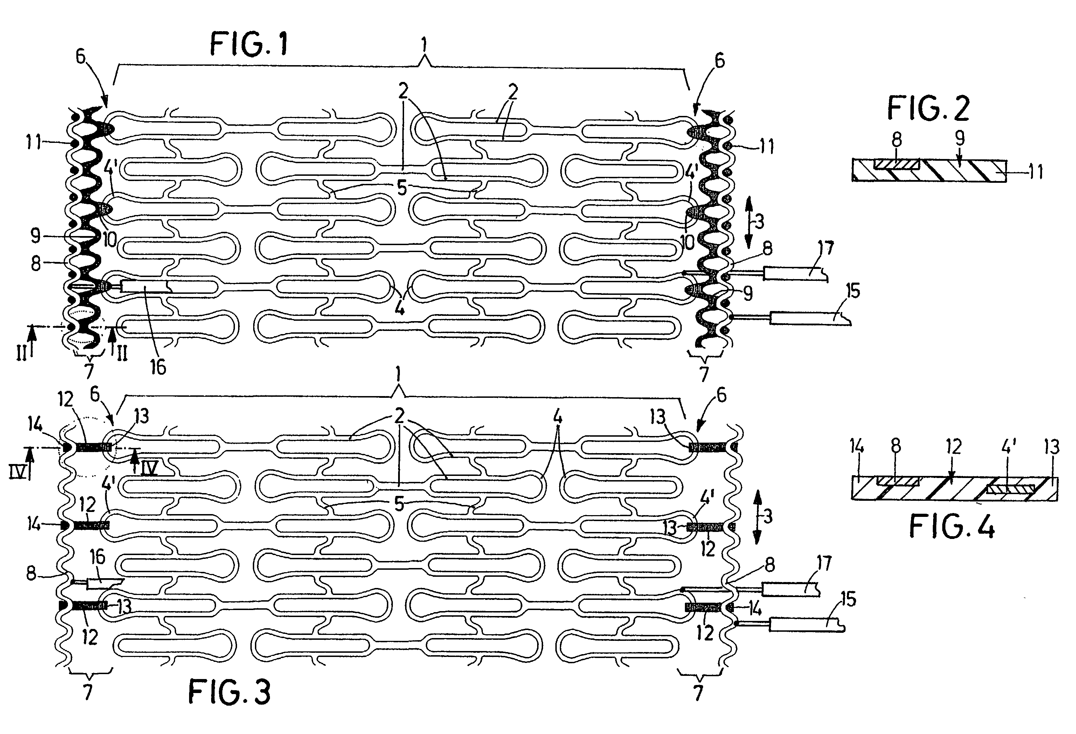

Bei der in Fig. 1 gezeigten Ausführungsform der Herzelektrodenanordnung ist eine zentrale, stentartige Defibrillatorelektrode 1 vorgesehen, die aus einer von Stents oder sogenannten Gefäßwandstützen bekannten, netzartigen Strebenstruktur besteht. Letztere weist sich verzweigende Längsstreben 2 auf, die im implantierten Zustand parallel zur Strömungsrichtung des Blutes im Gefäß liegen. Einstückig damit verbunden sind im wesentlichen in Peripherrichtung 3 verlaufende Querstreben, die einerseits als Bogenstreben 4 die Längsstreben 2 an ihren Enden verbinden. Andererseits verlaufen zwischen nebeneinanderliegenden Längsstreben 2 die als Verbindungsstreben 5 ausgebildeten Querstreben und stellen somit die netzartige Struktur her. Die Defibrillatorelektrode 1 ist insgesamt aus einem medizinisch verträglichen, dünnen Metallblech einstückig durch Stanzen, Laserstrahl- oder Wasserstrahlschneiden hergestellt.

An den beiden Längsenden 6 sind als Ganzes mit 7 bezeichnete Isolationszonen vorgesehen, über die die endseitigen Schrittmacherelektroden 8 angebunden sind. Diese laufen analog der Defibrillatorelektrode 1 ringartig in Peripherrichtung 3 um und weisen eine wellenförmige Gestalt auf. Sie sind ebenfalls aus einem dünnen Metallblech mit Hilfe der oben angegebenen Bearbeitungsverfahren ausgeschnitten. Wie aus Fig. 1 erkennbar ist, weisen die Schrittmacherelektroden 8 eine um Größenordnungen kleinere Oberfläche gegenüber der Defibrillatorelektrode auf.

Die Isolationszonen 7 sind in dem Ausführungsbeispiel gemäß Fig. 1 und 2 jeweils als durchgehende Isolationsbrücken 9 aus elektrisch isolierendem Polymermaterial gebildet. Jede dieser Isolationsbrücken 9 besteht dabei aus einem in Peripherrichtung 3 verlaufenden, wellenförmigen Polymerstrang. Die nach innen weisenden Wellenköpfe 10 des Stranges umschließen durch Vergießen dabei die außenstehenden Bogenstreben 4' der Defibrillatorelektrode 1. Die nach außen gewandten Wellenköpfe 11 der beiden Isolationsbrücken 9 sind analog durch Angießen an die nach innen weisenden Wellenköpfe der wellenförmigen Schrittmacherelektroden 8 mit diesen verbunden. Der die Isolationsbrücke 9 bildende Polymerstrang, der verglichen zu der stark vergrößerten Darstellung der beigefügten Figuren sehr filigran ist, kann durch Aufgießen eines Ringbandes und anschließendes Wegätzen sowie Polieren hergestellt werden.

Die in Fig. 3 und 4 dargestellte Version einer erfindungsgemäßen Elektrodenanordnung unterscheidet sich bezüglich der Ausgestaltung der Defibrillatorelektrode 1 und der beiden Schrittmacherelektroden 8 nicht von dem Ausführungsbeispiel gemäß Fig. 1 und 2. Insoweit kann auf die Beschreibung dieses Ausführungsbeispiels verwiesen werden. Übereinstimmende Bauteile sind dabei mit identischen Bezugszeichen in Fig. 3 und 4 versehen.

Als Unterschied zwischen den beiden Ausführungsbeispielen ist lediglich festzuhalten, daß bei der Elektrodenanordnung gemäß Fig. 3 und 4 die Isolationszone 7 jeweils aus einzelnen, über den Umfang der Elektrodenanordnung verteilten Brückenstreben 12 gebildet ist. Diese Brückenstreben 12 sind mit ihren Enden 13 mit den außenseitigen, am Rand gelegenen Bogenstreben 4' der Defibrillatorelektrode 1 vergossen. Mit dem zweiten Ende 14 ist jede Brückenstrebe 12 an einen nach innen weisenden Wellenkopf der wellenförmigen, umlaufenden Schrittmacherelektrode 8 angegossen.

Die Kontaktierung der Defibrillator- und Schrittmacherelektrode 1, 8 erfolgt in beiden Ausführungsbeispielen durch die elektrischen Zuleitungen 15, 16, 17. Diese sind in einem dünnen, nicht näher dargestellten Katheter geführt. Die elektrisch leitfähigen Seelen der elektrischen Zuleitungen 15, 16, 17 sollten - wie nicht näher dargestellt ist - aus sogenannten Seilen bestehen, die im Gegensatz zu den häufig bei Kathetern als Zuleitungen verwendeten Wendeln flexibler sind und einen geringeren Durchmesser besitzen. Dies ist insbesondere im Hinblick auf den Einsatz der erfindungsgemäßen Elektrodenanordnung in kleinen Blutgefäßen, wie dem Koronarsinus, wegen der geringeren Irritation der Gefäßwände und der Minimierung einer Behinderung des Blutflusses von großer Bedeutung. Diese Seile bestehen in der Regel aus einigen wenigen Litzen von sogenanntem Medizin-Stahl (MP 35 N) oder aus Edelstahllitzen mit Silberkern (DFT = Drawn Filed Tube).

Besonderes Augenmerk bezüglich der Kontaktierung muß auf die distale Schrittmacher-Elektrode - in den Fig. 1 und 3 also die linke Schrittmacher-Elektrode 8 - gelegt werden. Die Zuleitung 16 muß elektrisch isoliert über die gesamte Länge der stentartigen Defibrillatorelektrode 1 geführt werden. Hierfür sind drei Varianten denkbar, nämlich komplett außenliegend, komplett innenliegend oder eine durch die einzelnen Streben 4, 5 geflochtene Variante. In herstellungs- und implantationstechnischer Hinsicht ist der komplett außenliegenden Variante der Vorzug zu geben. Die komplett innenliegende Führung der Zuleitung ist nämlich problematisch, da diese Zuleitung beim Dilatieren der stentartigen Elektrodenanordnung mit einem Druck von 8 bis 12 bar beschädigt oder an ihrem Kontaktpunkt an der jeweiligen Elektrode davon abgelöst werden kann. Die Zuleitung kann damit störend in den Blutstrom geraten. Die geflochtene Variante ist hinsichtlich der elektrischen Isolation der Befestigungspunkte der Zuleitung an der Elektrode unproblematisch.

Beide Ausführungsformen sind in den Fig. 1 und 3 *im übrigen* im nicht dilatierten Zustand dargestellt. Beim Expandieren eines Ballonkatheters zur Dilatation werden die geschwungenen Verbindungsstreben 5 zwischen nebeneinanderliegenden Längsstreben 2 gestreckt, wodurch sich die Defibrillatorelektrode 1 aufweitet und an die Innenwand des Koronarsinus anlegen kann. Ein ähnlicher Reck- oder Streckeffekt erfolgt bei den wellenförmigen Schrittmacherelektroden 8 und den wellenförmigen Isolationsbrücken 9 beim Ausführungsbeispiel gemäß Fig. 1. Bei der Ausführungsform nach Fig. 3 werden bei der Dilatation die einzelnen Brückenstreben 12 voneinander entfernt.

Bezüglich der Isolationszonen ist abschließend darauf hinzuweisen, daß diese nicht unbedingt aus Polymermaterial, wie bei den Ausführungsformen gemäß den beiliegenden Fig. 1 bis 4, hergestellt sein muß. Es kann die gesamte Elektrodenanordnung auch einstückig aus einem elektrisch leitfähigen Material hergestellt werden, wonach die Isolationszonen 7 zwischen der Defibrillator- und den Schrittmacherelektroden 1, 8 durch Wärmebehandlung oder Ionenimplantation des Materials elektrisch isolierend gemacht werden. Damit ist eine besondere homogene Elektrodenstruktur erreichbar, was sich vorteilhaft auf das Einführverhalten und die Langzeitverträglichkeit der Elektrodenanordnung auswirkt.

## Patentansprüche

1. Dilatierbare Herzelektrodenanordnung zur Implantation insbesondere im Koronarsinus des Herzens mit
- einer stentartigen Defibrillatorelektrode (1) mit einer aufweitbaren, elektrisch leitfähigen Struktur,
- ringartigen oder ringabschnittartigen Schrittmacherelektroden (8), die
-- gegenüber der Defibrillatorelektrode (1) eine kleinere Oberfläche aufweisen,
-- an den einander abgewandten Enden (6) der Defibrillatorelektrode (1) angeordnet sind und
-- ebenfalls eine stentartig aufweitbare, elektrisch leitfähige Struktur aufweisen,
- Isolationszonen (7) jeweils zwischen den Schrittmacherelektroden (8) einerseits und der Defibrillatorelektrode (1) andererseits, sowie
- elektrischen Zuleitungen (15, 16, 17) für die Defibrillator- und Schrittmacherelektroden (1, 8).

2. Elektrodenanordnung nach Anspruch 1, **dadurch gekennzeichnet, daß** die Defibrillatorelektrode (1) aus einer netzartigen Strebenstruktur bestehend aus Längsstreben (2) und einstückig damit verbundenen, im wesentlichen in Peripherrichtung (3) verlaufenden Querstreben (4, 4', 5, 5') gebildet ist.

3. Elektrodenanordnung nach Anspruch 1 oder 2, **dadurch gekennzeichnet, daß** die Schrittmacherelektroden (8) jeweils mindestens eine in Peripherrichtung (3) umlaufende wellenförmige Strebe aufweisen.

4. Elektrodenanordnung nach einem der Ansprüche 1 bis 3, **dadurch gekennzeichnet, daß** die Isolationszonen durch Isolationsbrücken (9) aus elektrisch isolierendem Polymermaterial gebildet sind.

5. Elektrodenanordnung nach Anspruch 4, **dadurch gekennzeichnet, daß** die Isolationsbrücken (9) jeweils aus einem in Peripherrichtung verlaufenden, wellenförmigen, an die randseitigen Streben (4') der Defibrillator- und Schrittmacherelektroden (1, 8) angegossenen oder mit diesen vergossenen Polymerstrang gebildet sind.

6. Elektrodenanordnung nach Anspruch 4, **dadurch gekennzeichnet, daß** die Isolationsbrücken (9) aus einzelnen, über den Umfang der Elektrodenanordnung verteilten Brückenstreben (12) gebildet sind, die mit ihren einander abgewandten Enden (13, 14) an die randseitigen Streben (4') der Defibrillator- und an die Schrittmacherelektrode (1, 8) angegossen oder mit diesen vergossen sind.

7. Elektrodenanordnung nach einem der Ansprüche 1 bis 3, **dadurch gekennzeichnet, daß** die gesamte Elektrodenanordnung einstückig aus einem elektrisch leitfähigen Material hergestellt ist, wobei die Isolationszonen zwischen Defibrillator- und Schrittmacherelektroden durch Wärmebehandlung oder Ionenimplantation des Materials elektrisch isolierend gemacht sind.

8. Elektrodenanordnung nach einem der Ansprüche 1 bis 7, **dadurch gekennzeichnet, daß** die beiden ringförmigen Schrittmacherelektroden (8) gemeinsam eine bipolare Schrittmacherelektrodenanordnung bilden.

9. Elektrodenanordnung nach einem der Ansprüche 1 bis 8, **dadurch gekennzeichnet, daß** die Seelen der elektrischen Zuleitungen (15, 16, 17) als dünne Seile ausgebildet sind.

10. Elektrodenanordnung nach einem der Ansprüche 1 bis 9, **dadurch gekennzeichnet, daß** mindestens die die distale Schrittmacher-Elektrode (8) kontaktierende Zuleitung (16) komplett außenliegend an der Herzelektrodenanordnung vorbeigeführt ist.

## Revendications

1. Dispositif d'électrode dilatable pour implantation en particulier dans le sinus coronaire du coeur avec
- une électrode de défibrillateur (1) de type stent avec une structure extensible conductrice d'électricité,
- des électrodes de pacemaker (8) annulaires ou de type à section annulaire, qui
-- présentent par rapport à l'électrode de défibrillateur (1) une surface plus petite,
-- sont placées sur des extrémités (6) détournées l'une de l'autre de l'électrode de défibrillateur (1), et
-- présentent aussi une structure extensible de type stent conductrice d'électricité,
- des zones d'isolation (7) placées chacune entre les électrodes de pacemaker (8) d'une part et l'électrode de défibrillateur (1) d'autre part, ainsi que
- des alimentations électriques (15, 16, 17) pour les électrodes de défibrillateur et pour les électrodes de pacemaker (1, 8).

2. Dispositif d'électrode selon la revendication 1, **caractérisé en ce que** l'électrode de défibrillateur (1) est constituée d'une structure de montants de type filet constituée de montants longitudinaux (2) et de montants transversaux (4, 4', 5, 5') liés en une pièce aux montants longitudinaux, s'étendant globalement en direction périphérique (3).

3. Dispositif d'électrode selon la revendication 1 ou 2, **caractérisé en ce que** les électrodes de pacemaker (8) présentent au moins un montant en forme d'onde s'étendant en direction périphérique (3).

4. Dispositif d'électrode selon l'une des revendications 1 à 3, **caractérisé en ce que** les zones d'isolation sont constituées de ponts d'isolation (9) en matériau polymère isolé électriquement.

5. Dispositif d'électrode selon la revendication 4, **caractérisé en ce que** les ponts d'isolation (9) sont constitués d'un cordon de polymère s'étendant en direction périphérique, en forme d'onde, coulé sur le montant (4') de côté de bord des électrodes de défibrillateur et de pacemaker (1, 8) ou scellé avec celles-ci.

6. Dispositif d'électrode selon la revendication 4, **caractérisé en ce que** les ponts d'isolation (9) sont formés de montants de ponts (12) individuels répartis sur la circonférence du dispositif d'électrode, qui sont coulés avec leurs extrémités (13, 14) détournées l'une de l'autre sur le montant (4') de côté latéral des électrodes de défibrillateur ou de pacemaker (1, 8) ou scellés avec celles-ci.

7. Dispositif d'électrode selon l'une des revendications 1 à 3, **caractérisé en ce que** l'ensemble du dispositif d'électrode est fabriqué en une pièce dans un matériau conducteur d'électricité, les zones d'isolation entre les électrodes de défibrillateur et de pacemaker étant rendues isolantes électriquement par traitement à la chaleur ou implantation ionique du matériau.

8. Dispositif d'électrode selon l'une des revendications 1 à 7, **caractérisé en ce que** les deux électrodes de pacemaker (8) annulaires forment ensemble un dispositif d'électrode de pacemaker bipolaire.

9. Dispositif d'électrode selon l'une des revendications 1 à 8, **caractérisé en ce que** les âmes des alimentations électriques (15, 16, 17) sont constituées comme des câbles fins.

10. Dispositif d'électrode selon l'une des revendications 1 à 9, **caractérisé en ce qu'**au moins l'alimentation (16) raccordant l'électrode de pacemaker (8) distale passe à côté du dispositif d'électrode cardiaque en étant complètement à l'extérieur.

## Claims

1. A dilatable cardiac electrode arrangement for implantation in particular in the coronary sinus of the heart, comprising
- a defibrillator electrode (1) of the type of a stent having an expandable, electrically conductive structure;
- pacemaker electrodes (8) in the form of a circle or a section of a circle,
-- which have a smaller surface as compared to the defibrillator electrode (1),
-- which are disposed on the ends (6), facing away from each other, of the defibrillator electrode (1), and
-- which also have an electrically conductive structure which is expandable in the way of a stent;
- insulation zones (7) between the pacemaker electrodes (8) on the one hand and the defibrillator electrode (1) on the other; and
- electric lines (15, 16, 17) for the defibrillator and pacemaker electrodes (1,8).

2. An electrode arrangement according to claim 1, **characterized in that** the defibrillator electrode (1) consists of a reticular rib structure of lengthwise ribs (2) and crosswise ribs (4, 4', 5, 5') which are integrally connected therewith and extend substantially in the peripheral direction (3).

3. An electrode arrangement according to claim 1 or 2, **characterized in that** the pacemaker electrodes (8) comprise at least one wavy rib encircling in the peripheral direction (3).

4. An electrode arrangement according to one of claims 1 to 3, **characterized in that** the insulation zones are formed by insulation bridges (9) of electrically insulating polymeric material.

5. An electrode arrangement according to claim 4, **characterized in that** the insulation bridges (9) are formed by a wavy polymer strand which extends in the peripheral direction and is joined to the marginal ribs (4') of the defibrillator and pacemaker electrodes (1, 8) by casting or is cast thereon.

6. An electrode arrangement according to claim 4, **characterized in that** the insulation bridges (9) are formed by individual bridging ribs (12), which are distributed over the circumference of the electrode arrangement and the ends (13, 14), facing away from each other, of which are joined by casting to the marginal ribs (4') of the defibrillator electrode and to the pacemaker electrode (1, 8) or are cast thereon.

7. An electrode arrangement according to one of claims 1 to 3, **characterized in that** the entire electrode arrangement is manufactured integrally from electrically conductive material, the insulation zones between defibrillator and pacemaker electrodes being rendered electrically insulating by heat treatment or ion implantation of the material.

8. An electrode arrangement according to one of claims 1 to 7, **characterized in that** the two annular pacemaker electrodes (8) jointly form a bipolar pacemaker electrode arrangement.

9. An electrode arrangement according to one of claims 1 to 8, **characterized in that** the cores of the electric lines (15, 16, 17) are thin cables.

10. An electrode arrangement according to one of claims 1 to 9, **characterized in that** at least the line (16) that contacts the distal pacemaker electrode (8) is guided completely outside along the cardiac electrode arrangement.
